# EUROPEAN PATENT SPECIFICATION

(11) **EP 1 285 655 B1**
(45) Date of publication and mention of the grant of the patent: **30.05.2007**
(21) Application number: 01919870.4
(22) Date of filing: 10.04.2001
(51) Int. Cl.: A61K 31/4422, A61K 31/4427, A61K 31/4439, A61K 31/444, A61K 31/675, A61K 31/443, A61K 31/4545, A61K 47/02, C07D 211/90, C07D 211/92, C07D 401/06, C07D 405/06, C07F 9/59, A61K 9/20

(54) **STABILIZED PHARMACEUTICAL COMPOSITIONS CONTAINING THE CALCIUM CHANNEL BLOCKER AZELNIDIPINE**
STABILISIERTE PHARMAZEUTISCHE ZUSAMMENSTELLUNGEN DIE DEN CALZIUMKANALBLOCKER AZELNIDIPINE ENTHALTEN
COMPOSITIONS PHARMACEUTIQUES STABILISEES CONTENANT LE BLOQUEUR DES CANAUX CALCIUM AZELNIDIPINE

(30) Priority: 11.04.2000 JP 2000108850
(43) Date of publication of application: 26.02.2003
(73) Proprietor: Sankyo Company, Limited, Tokyo 103-8426 (JP); Ube Industries, Ltd., Ube-Shi, Yamaguchi 755-8633 (JP)
(72) Inventor: WAKIYAMA, Naoki c/o SANKYO COMPANY, LIMITED, Tokyo 140-8710 (JP); USUI, Fusao c/o SANKYO COMPANY, LIMITED, Tokyo 140-8710 (JP); NISHIMURA, Kenji c/o SANKYO COMPANY, LIMITED, Tokyo 140-8710 (JP)
(74) Representative: Gibson, Christian John Robert
(86) International application number: PCT/JP2001/003087
(87) International publication number: WO 2001/076598

(56) References cited:
- EP-A- 0 249 587
- EP-A2- 0 521 310
- EP-A2- 0 884 054
- WO-A-99/27912
- JP-A- 10 167 966
- US-A- 4 665 081
- US-A- 4 794 111
- US-A- 5 762 950

## Description

### [Technical field]

The present invention relates to a stabilized pharmaceutical composition containing a calcium blocker, which is azelnidipine or a pharmacologically acceptable salt thereof.

### [Background of the invention]

Calcium blockers are well known as antihypertensive agents, which can exist in a lot of formulations and are commercially available (for example, USP 3,485,847, USP 3,985,758, USP 4,572,909 and the like). WO 99/27912 discloses the incorporation of greasy/oily drugs, such as the calcium blocker felodipine, with a greasy/oily substance into hydroxyapatite. US-A-5,762,950 discloses drug delivery systems comprising a drug such as the calcium blocker nifedipine, and a bioceramic comprising hydroxyapatite and a bioactive glass/ceramic. EP-A-0 249 587 relates to extended release preparations comprising drugs such as nifedipine and felodipine, and a nonionic solubilizer. US-A-4,665,081 discloses solid preparations of nifedipine, comprising casein and one or more inorganic excipients such as magnesium silicate, magnesium carbonate, aluminium hydroxide, magnesium oxide, magnesium aluminate metasilicate, hydrotalcite and magnesium aluminium hydroxide. JP-A-10 167 966 relates to solid preparations comprising a 1,4-dihydropyridine derivative such as nitrendipene, and magnesium carbonate. EP-A-0 521 310 relates to solid dispersion tablets comprising a 1,4-dihydropyridine derivative such as nifedipine, nisoldipine, nicardipine, nimodipine, nilvadipine, nitrendipene and manidipine, and hydroxypropyl methyl cellulose phthalate. US-A-4,794,111 discloses compositions comprising a synergistically effective mixture of a dihydropyridine derivative selected from nisoldipine, nimodipine and nitrendipene, and a beta-blocker. EP-A-0 884 054 relates to compositions comprising a synergistically effective combination of enalapril and nitrendipine. These formulations, however, are not always satisfactory in their stability such as their storage stability. A pharmaceutical composition having excellent stability such as storage stability has been desired.

The inventors have made a great effort on the study of pharmaceutical compositions containing calcium blockers for a long period. They have found that a pharmacologically acceptable alkaline material is added to a calcium blocker to afford a pharmaceutical composition having excellent stability such as storage stability.

The present invention relates to a stabilized pharmaceutical composition containing a calcium blocker.

### [Disclosure of the invention]

The present invention is a pharmaceutical composition containing a calcium blocker which is azelnidipine or a pharmacologically acceptable salt thereof and a pharmacologically acceptable alkaline material which is added to an extent such that an aqueous solution or dispersion solution of said pharmaceutical composition containing a calcium blocker has a pH of at least 8.

Planar chemical structured of azelnidipine is shown below.

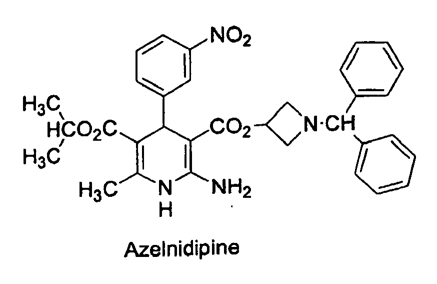

Azelnidipine is 2-amino-3-(1-diphenylmethyl-3-azetidinyloxycarbonyl)-5-isopropoxycarbonyl-6-methyl-4-(3-nitrophenyl)-1,4-dihydropyridine disclosed in USP 4,772,596, Japanese patent publication No. Sho 63-253082 and the like.

When azelnidipine has asymmetric carbon(s) and/or double bond(s), they can exist as optically active isomers, geometrical isomers and/or ring structural isomers. The present invention encompasses the individual optical, geometrical and structural isomers and mixtures thereof.

Pharmacologically acceptable salts of azelnidipine are acid addition salts, for example, hydrohalogenic acid salts such as hydrofluoride, hydrochloride, hydrobromide and hydroiodide; nitrate; perchlorate; sulfate; phosphate; carbonate; alkylsulfonates having 1 to 6 carbons optionally substituted with fluorine atom(s) such as methanesulfonates, trifluoromethanesulfonate, ethanesulfonate, pentafluoroethanesulfonate, propanesulfonate, butanesulfonate, pentanesulfonate and hexanesulfonate; arylsulfonates having 6 to 10 carbons such as benzenesulfonate and p-toluenesulfonate; carboxylic acid salts such as acetate, propionate, butyrate, benzoate, fumarate, maleate, succinate, citrate, tartrate, oxalate and malonate; or amino acid salts such as glutamate and aspartate. Preferred salts are hydrochlorides.

Azelnidipine or salts thereof can exist as hydrates and this invention encompasses such hydrates.

The pharmaceutical compositions of this invention contain 0.5 to 60 parts of a calcium blocker by weight based on 100 parts by weight of said composition, preferably 1 to 30 parts by weight.

The pharmacologically acceptable alkaline materials employed in this invention with which an aqueous solution or dispersion solution of said pharmaceutical composition can be adjusted to at least pH 8, are pharmaceutically acceptable alkaline materials known to those skilled in the art and include alkaline materials which are soluble, slightly soluble or substantially insoluble in water. Examples of such alkaline materials are alkali metal hydroxides such as lithium hydroxide, sodium hydroxide and potassium hydroxide; alkaline earth metal hydroxides such as magnesium hydroxide, calcium hydroxide and barium hydroxide; aluminium hydroxide; alkali metal carbonates such as lithium carbonate, sodium carbonate and potassium carbonate; alkaline earth metal carbonates such as magnesium carbonate, calcium carbonate and barium carbonate; alkali metal hydrogencarbonates such as lithium hydrogencarbonate, sodium bicarbonate and potassium hydrogencarbonate; di-alkali metal phosphates such as disodium phosphate and dipotassium phosphate; di-alkaline earth metal phosphates such as dimagnesium phosphate, dicalcium phosphate and dibarium phosphate; trialkali metal phosphates such as trisodium phosphate and tripotassium phosphate; alkaline earth metal oxides such as magnesium oxide and calcium oxide; aluminum oxide; alkali metal silicates such as sodium silicate and potassium silicate; alkaline earth metal silicates such as magnesium silicate and calcium silicate; silicic acid-aluminum complex compounds such as silicic acid-alumina; aluminum-magnesium complex compounds such as magnesium aluminosilicate and magnesium aluminometasilicate; or mixtures thereof. Preferred alkaline materials are alkali metal carbonates, alkaline earth metal carbonates, alkali metal hydrogencarbonates, alkaline earth metal oxides, alkali metal silicates, aluminum-magnesium complex compounds, or mixtures thereof. More preferred alkali materials are sodium carbonate, magnesium carbonate, calcium carbonate, sodium bicarbonate, magnesium oxide, calcium oxide, magnesium silicate, calcium silicate, magnesium aluminosilicate and magnesium aluminometasilicate; or mixtures thereof. Most preferred alkali materials are sodium carbonate, sodium bicarbonate, calcium silicate, magnesium aluminosilcate and magnesium aluminometasilcate; or mixtures thereof (particularly, mixtures of sodium carbonate and magnesium aluminometasilicate aluminate or sodium bicarbonate and magnesium aluminometasilicate (in a ratio 1/20 to 1/2)).

The amount of the alkaline material is not particularly limited provided that an aqueous solution or dispersion solution of said pharmaceutical composition can be adjusted to at least pH 8 with said alkaline material. The preferred amount of the alkaline material is from 1 to 70 parts by weight based on 100 parts by weight of said composition, preferably 5 to 50 parts by weight.

The preferred pH of the aqueous solution or dispersion solution of said pharmaceutical composition is between 8 and 12, more preferably between 9 to 11. The pH of the aqueous solution or dispersion solution of said pharmaceutical composition is determined by measurement of the solution on a pH meter which solution is obtained by 1) dissolution or dispersion of a ten-fold amount of a unit dosage of said pharmaceutical composition (for example one tablet, or one capsule) in 100 ml distilled water as described in the Japanese Pharmacopoeia, 2) centrifugation of the mixture, and 3) filtration of the supernatant.

When said pharmaceutical composition absorbs water or a small amount of water is added to said pharmaceutical composition, the pH (micro-pH) of the surroundings of the particles of said pharmaceutical composition can be adjusted to at least 8 with the pharmacologically acceptable alkaline material which is one component in this invention.

The pharmaceutical composition of this invention may appropriately contain pharmaceutically acceptable additives. Examples of such additives are excipients (for example, sugar derivatives such as lactose, sucrose, glucose, mannitol and sorbitol; starch derivatives such as corn starch, potato starch, α-starch, dextrin, carboxymethyl starch and sodium carboxymethyl starch; gelatinized starch; cellulose derivatives such as crystalline cellulose, methylcellulose, hydroxypropylcellulose, lower substituted hydroxypropylcellulose, hydroxypropylmethylcellulose, carboxymethylcellulose, calcium carboxymethylcellulose, cross-linked carboxymethylcellulose and cross-linked sodium carboxymethylcellulose; acacia; dextran; pullulan; silicate derivatives such as light silicic acid anhydride, silicic acid hydrate, synthetic aluminum silicate and magnesium aluminometasilicate; phosphate derivatives such as dicalcium phosphate; chloride salt derivatives such as sodium chloride; carbonate derivatives such as calcium carbonate; sulfate derivatives such as calcium sulfate; or mixtures thereof; preferably sugar derivatives, cellulose derivatives or mixtures thereof, more preferably mannitol, crystalline cellulose or mixtures thereof), binding agents (for example, compounds illustrated above as excipients, gelatin, polyvinylpyrrolidone, macrogol, or mixtures thereof; preferably cellulose derivatives or mixtures thereof; more preferably hydroxypropylmethyl cellulose), disintegrating agents (for example, the compounds illustrated above as excipients; cross-linked polyvinylpyrrolidone; or mixtures thereof; preferably cellulose derivatives or mixtures thereof; more preferably lower substituted hydroxypropylmethylcellulose, calcium carboxymethylcellulose or mixtures thereof), lubricating agents (for example, stearic acid; metal stearates such as calcium stearate and magnesium stearate; metal benzoates such as sodium benzoate; waxes such as beeswax and spermaceti; boric acid; glycol; carboxylic acids such as fumaric acid and adipic acid; metal sulfates such as sodium sulfate; Leucine; metal lauryl sulfates such as sodium lauryl sulfate and magnesium lauryl sulfate; the silicate derivatives illustrated above as excipients; the cellulose derivatives illustrated above as excipients; hydrogenated vegetable oil; carnauba wax; sucrose esters of fatty acids; or mixtures thereof; preferably metal stearates, silicate derivatives, or mixtures thereof and more preferably calcium stearate, magnesium stearate, silicic acid anhydride, or mixtures thereof), stabilizing agents (for example, benzoic acid, metal benzoates such as sodium benzoate; paraoxybenzoates such as methylparaben and propylparaben; alcohols such as chlorobutanol, benzyl alcohol and phenylethyl alcohol; benzalkonium chloride; phenol derivatives such as phenol or cresol; thimerosal; acetic anhydride; sorbic acid or mixtures thereof; preferably metal benzoates, paraoxybenzoates, or mixtures thereof; more preferably sodium benzoate, methylparaben, propylparaben, or mixtures thereof), fluidizing agents (for example, the silicate derivatives illustrated above as excipients; talc; or mixtures thereof; preferably light silicic acid anhydride, talc or mixtures thereof), surface activating agents (for example, polysorbates such as polysorbate 80; polyoxyethylene hydrogenated castol oils such as polyoxyethylene hydrogenated castol oil 60; sorbitan esters of fatty acids; sucrose esters of fatty acids; polyoxyethylenepolyoxypropylenglycols; polyoxyethylene ethers of fatty acids; polyoxyl stearates; or mixtures thereof; preferably polysorbate 80, polyoxyethylene hydrogenated castol oil 60 or mixtures thereof), coloring agents, anti-oxidating agents, corrigents (for example, sweetening, souring and flavoring agents which are conventionally used), or diluents.

Additives employed in this invention and the amount of said additives will vary with tablets, capsules, and other dosage forms, and they can be determined by techniques known to those skilled in the art. Tablets may usually contain binder(s) in an amount of 1 to 10 parts by weight (preferably 3 to 5 parts), disintegrant(s) in an amount of 1 to 40 parts by weight (preferably 5 to 30 parts), lubricant(s) in an amount of 0.1 to 10 parts by weight (preferably 0.5 to 3 parts) and fluidizing agent(s) in an amount of 1 to 10 parts by weight (preferably 2 to 5) based on 100 parts by weight of said pharmaceutical composition.

Azelnidipine, which is an active ingredient of this invention, can be easily prepared according to techniques known to those skilled in the art (for example USP 4,772,596 and Japanese patent publication No. Sho 63-253082).

The pharmaceutical compositions of the present invention can be prepared easily by using azelnidipine or salts thereof, alkaline materials and pharmaceutically acceptable additives in a known manner (for example, procedures such as mixing and kneading with water and wet granulation, etc.). Formulations such as tablets, capsules and granules, for example, can be prepared as follows. To the alkaline materials placed in a high shear granulator is added surfactant(s) as needed, and then azelnidipine or a salt thereof, fillers, binders and disintegrants are furthermore added with mixing. In some cases, other kinds of alkaline materials are also added as needed. Subsequently, an aqueous solution of the binder(s) is added to the mixture obtained to prepare a wet mass in the high shear granulator. In the preparation of tablets and capsules, the wet mass obtained is dried in a fluid bed dryer, and the dried mass obtained is cut by a cutting mill and passed through a screen. The desired tablets or capsules can be prepared by mixing the screened granules and lubricant(s) with a V-shaped blender and then tableting or filling the resulting mixture into capsules, respectively. On the other hand, in the preparation of granules, the wet mass obtained above is extruded using an extrusion granulator to prepare wet granules, which are then dried using an air-through tray dryer. The desired granules can be obtained by cutting the dried granules obtained using the cutting mill and then passing through a screen.

The present invention is described in more detail by Examples.

### Best mode for carrying out the invention

### (Example 1) Tablets 1

The desired tablets were prepared using the components, the quantity of each of which is listed in the formula shown in Table 1, as follows.

To light magnesium aluminometasilicate (Grade FL2) placed in a high shear granulator was added polysorbate 80 with stirring, and then Azelnidipine, crystalline cellulose, D-mannitol, low substituted hydroxypropylcellulose and sodium bicarbonate were added successively with mixing. Subsequently, an aqueous hydroxypropylcellulose solution was added to the mixture to prepare a wet mass, which was dried in a fluid bed dryer into which inlet air at 90°C was supplied continuously until the temperature of the exhausted air from the dryer went up to 55°C. The dried mass obtained was cut by a cutting mill and passed through a screen of 1.0-mm meshes. The desired tablets were prepared by mixing the screened granules and magnesium stearate for 10 min using a V-shaped blender and then compressing the resulting mixture using a tableting machine with a punch of 8.0-mm diameter.

In each of Examples 1 - 5 and Reference example 1, 8 mg of Azelnidipine was used.

**(Table 1)**

| Component | Quantity |
|---|---|
| | (Weight percentage) |
| Azelnidipine | 5 |
| Crystalline cellulose | 5 |
| D-mannitol | 8 |
| Low substituted hydroxypropylcellulose | 15 |
| Light magnesium aluminometasilicate | 45 |
| Sodium bicarbonate | 3 |
| Hydroxypropylcellulose | 3 |
| Polysorbate 80 | 15 |
| Magnesium stearate | 1 |
| Total | 100 |

A suitable amount of this formulation was pulverized in an agate mortar and passed through a sieve with 20 meshes. Subsequently, 1000 mg of the powder obtained (corresponding to five tablets) was placed in a centrifuge tube and after the addition of 50 ml of purified water as defined by The Pharmacopoeia of Japan, the resulting mixture was shaken for 20 min using a shaker. After shaking, the resulting suspension was centrifuged at 3000 rpm for 10 min and the supernatant obtained was passed through a filter with a pore size of 0.45-µm, and then the pH value of the filtrate was measured with a pH meter. The pH value of the solution obtained was 9.5.

When this formulation was stored at 25°C under lightproof and water-resistant conditions, 98% of the active ingredient in this formulation was detected as unaltered even after storage for 36 months.

### (Example 2) Tablets 2

The desired tablets were prepared using the components, the quantity of each of which is listed in the formula shown in Table 2, as follows.

To a mixture of light magnesium aluminometasilicate (Grade FL2) and light silicic acid anhydride in a high shear granulator was added polysorbate 80 with stirring, and then Azelnidipine, crystalline cellulose, D-mannitol, low substituted hydroxypropylcellulose, carboxymethylcellulose calcium (carmellose calcium) and sodium bicarbonate were added successively with mixing. Subsequently, an aqueous hydroxypropylcellulose solution was added to the mixture to prepare a wet mass, which was dried in a fluid bed dryer into which inlet air at 90°C was supplied continuously until the temperature of the exhausted air from the dryer went up to 55°C. The dried mass obtained was cut by a cutting mill and passed through a screen of 1.0-mm meshes. The desired tablets were prepared by mixing the screened granules and magnesium stearate for 10 min using a V-shaped blender and then compressing the resulting mixture using a tableting machine with a punch of 8.0-mm diameter.

**(Table 2)**

| Component | Quantity |
|---|---|
| | (Weight percentage) |
| Azelnidipine | 5 |
| Crystalline cellulose | 5 |
| D-mannitol | 15 |
| Low substituted hydroxypropylcellulose | 15 |
| Carmellose calcium | 6 |
| Light magnesium aluminometasilicate | 25 |
| Light silicic acid anhydride | 6 |
| Sodium bicarbonate | 5 |
| Hydroxypropylcellulose | 5 |
| Polysorbate 80 | 12 |
| Magnesium stearate | 1 |
| Total | 100 |

The pH value of this formulation was measured in a similar manner to that mentioned in Example 1. The pH value of the solution obtained was 10.0.
When this formulation was stored at 25°C under lightproof and water-resistant conditions, 99% of the active ingredient in this formulation was detected as unaltered even after storage for 36 months.

### (Example 3) Capsules 1

The desired capsules were obtained by preparing a mixture of components, the quantity of each of which is listed in the formula shown in Table 2, in a similar manner to that mentioned in Example 2 and then filling a defined amount of the resulting mixture into each No. 3 capsule.

The pH value of this formulation was measured in a similar manner to that mentioned in Example 1. The pH value of the solution obtained was 10.0.

When this formulation was stored at 25°C under lightproof and water-resistant conditions, 98% of the active ingredient in this formulation was detected as unaltered even after storage for 36 months.

### (Example 4) Tablets 3

The desired tablets were prepared using sodium carbonate instead of sodium bicarbonate listed in the formula in Table 2 in a similar manner to that mentioned in Example 2.

The pH value of this formulation was measured in a similar manner to that mentioned in Example 1. The pH value of the solution obtained was 11.0.

When this formulation was stored at 25°C under lightproof and water-resistant conditions, 95% of the active ingredient in this formulation was detected as unaltered even after storage for 36 months.

### (Example 5) Tablets 4

The desired tablets were prepared using the components, the quantity of each of which is listed in the formula shown in Table 3, as follows.

To calcium silicate placed in a high shear granulator was added polysorbate 80 with stirring, and then Azelnidipine, D-mannitol and low substituted hydroxypropylcellulose were added successively with mixing. Subsequently, an aqueous hydroxypropylcellulose solution was added to the mixture to prepare a wet mass, which was dried in a fluid bed dryer into which inlet air at 90°C was supplied continuously until the temperature of the exhausted air from the dryer went up to 55°C. The dried mass obtained was cut by a cutting mill and passed through a screen of 1.0-mm meshes. The desired tablets were prepared by mixing the screened granules and magnesium stearate for 10 min with a V-shaped blender and then compressing the resulting mixture using a tableting machine with a punch of 8.0-mm diameter.

**(Table 3)**

| Component | Quantity |
|---|---|
| | (Weight percentage) |
| Azelnidipine | 5 |
| D-mannitol | 34 |
| Low substituted hydroxypropylcellulose | 20 |
| Calcium silicate | 20 |
| Hydroxypropylcellulose | 5 |
| Polysorbate 80 | 15 |
| Magnesium stearate | 1 |
| Total | 100 |

The pH value of this formulation was measured in a similar manner to that mentioned in Example 1. The pH value of the solution obtained was 9.3.

When this formulation was stored at 25°C under lightproof and water-resistant conditions, 97% of the active ingredient in this formulation was detected as unaltered even after storage for 36 months.

### (Reference example 1) Tablets A

The desired tablets were prepared using the components, the quantity of each of which is listed in the formula shown in Table 4, as follows.

Azelnidipine, D-mannitol and low substituted hydroxypropylcellulose were mixed in a high shear granulator, and then polysorbate 80 was further added with mixing. Subsequently, an aqueous hydroxypropylcellulose solution was added to the mixture to prepare a wet mass, which was dried in a fluid bed dryer into which inlet air at 90°C was supplied continuously until the temperature of the exhausted air from the dryer went up to 55°C. The dried mass obtained was cut by a cutting mill and passed through a screen of 1.0-mm meshes. The desired tablets were prepared by mixing the screened granules and magnesium stearate for 10 min with a V-shaped blender and then compressing the resulting mixture using a tableting machine with a punch of 8.0-mm diameter.

**(Table 4)**

| Component | Quantity |
|---|---|
| | (Weight percentage) |
| Azelnidipine | 5 |
| D-mannitol | 57 |
| Low substituted hydroxypropylcellulose | 20 |
| Hydroxypropylcellulose | 5 |
| Polysorbate 80 | 12 |
| Magnesium stearate | 1 |
| Total | 100 |

The pH value of this formulation was measured in a similar manner to that mentioned in Example 1. The pH value of the solution obtained was 7.4.

When this formulation was stored at 25°C under lightproof and water-resistant conditions, 70 % of the active ingredient in this formulation was detected as unaltered after storage for 36 months.

### Possibility of industrial use

The pharmaceutical compositions of this invention exhibit excellent storage stability, rapid absorption through the intestinal tract and can be prepared by an easy wet granulation method. These pharmaceutical compositions, therefore, are useful compositions as a medical formulation.

## Claims

1. A pharmaceutical composition containing a calcium blocker which is azelnidipine or a pharmacologically acceptable salt thereof and a pharmacologically acceptable alkaline material which is an alkali metal hydroxide, an alkaline earth metal hydroxide, an aluminum hydroxide, an alkali metal carbonate, an alkaline earth metal carbonate, an alkali metal hydrogencarbonate, a di-alkali metal phosphate, a di-alkaline earth metal phosphate, a tri-alkali metal phosphate, an alkaline earth metal oxide, aluminum oxide, an alkali metal silicate, an alkaline earth metal silicate, a silicic acid-aluminum complex compound, an aluminum-magnesium complex compound, or a mixture thereof, wherein the alkaline material is added to an extent such that an aqueous solution or dispersion solution of said pharmaceutical composition containing a calcium blocker has a pH of at least 8, as determined by measurement with a pH meter of a solution obtained by (i) forming a mixture by dissolution or dispersion of a ten-fold amount of a unit dosage of said pharmaceutical composition in 100 ml distilled water as described in the Japanese Pharmacopoeia, (ii) forming a supernatant by centrifugation of said mixture, and (iii) forming said solution by filtration of said supernatant.

2. A pharmaceutical composition according to claim 1 wherein the alkaline material is an alkali metal carbonate, an alkaline earth metal carbonate, an alkali metal hydrogencarbonate, an alkaline earth metal oxide, an alkali metal silicate, an alkaline earth metal silicate, an aluminum-magnesium complex compound, or a mixture thereof.

3. A pharmaceutical composition according to claim 2 wherein the alkaline material is sodium carbonate, magnesium carbonate, calcium carbonate, sodium bicarbonate, magnesium oxide, calcium oxide, magnesium silicate, calcium silicate, magnesium aluminosilicate, magnesium aluminometasilicate, or a mixture thereof.

4. A pharmaceutical composition according to claim 3 wherein the alkaline material is sodium carbonate, sodium bicarbonate, calcium silicate, magnesium aluminosilicate, magnesium aluminometasilicate, or a mixture thereof.

5. A pharmaceutical composition according to claim 4 wherein the alkaline material is a mixture of sodium carbonate and magnesium aluminometasilicate, or a mixture of sodium bicarbonate and magnesium aluminometasilicate.

6. A pharmaceutical composition according to claims 1 to 5 wherein the pH of an aqueous solution or dispersion solution of said pharmaceutical composition is between 8 and 12.

7. A pharmaceutical composition according to claim 6 wherein the pH of an aqueous solution or dispersion solution of said pharmaceutical composition is between 9 and 11.

## Patentansprüche

1. Pharmazeutische Zusammensetzung, enthaltend einen Calciumblocker, welcher Azelnidipin oder ein pharmakologisch verträgliches Salz davon ist, und ein pharmakologisch verträgliches alkalisches Material, welches ein Alkalimetallhydroxid, ein Erdalkalimetallhydroxid, ein Aluminiumhydroxid, ein Alkalimetallcarbonat, ein Erdalkalimetallcarbonat, ein Alkalimetallhydrogencarbonat, ein Dialkalimetallphosphat, ein Dierdalkalimetallphosphat, ein Trialkalimetallphosphat, ein Erdalkalimetalloxid, Aluminiumoxid, ein Alkalimetallsilicat, ein Erdalkalimetallsilicat, eine Kieselsäure-Aluminium-Komplexverbindung, eine Aluminium-Magnesium-Komplexverbindung oder ein Gemisch davon ist, wobei das alkalische Material in einem derartigen Ausmaß hinzugegeben wird, daß eine wässerige Lösung oder Dispersionslösung der pharmazeutischen Zusammensetzung, enthaltend einen Calciumblocker, einen pH von mindestens 8, wie durch Messung mit einem pH-Meter einer Lösung, erhalten durch (i) Erzeugen eines Gemisches durch Auflösung oder Dispergierung einer zehnfachen Menge einer Einheitsdosierung der pharmazeutischen Zusammensetzung in 100 ml destilliertem Wasser, wie beschrieben in der japanischen Pharmakopöe, (ii) Erzeugen eines Überstands durch Zentrifugation des Gemisches und (iii) Erzeugen der Lösung durch Filtration des Überstands, bestimmt, hat.

2. Pharmazeutische Zusammensetzung nach Anspruch 1, wobei das alkalische Material ein Alkalimetallcarbonat, ein Erdalkalimetallcarbonat, ein Alkalimetallhydrogencarbonat, ein Erdalkalimetalloxid, ein Alkalimetallsilicat, ein Erdalkalimetallsilicat, eine Aluminium-Magnesium-Komplexverbindung oder ein Gemisch davon ist.

3. Pharmazeutische Zusammensetzung nach Anspruch 2, wobei das alkalische Material Natriumcarbonat, Magnesiumcarbonat, Calciumcarbonat, Natriumbicarbonat, Magnesiumoxid, Calciumoxid, Magnesiumsilicat, Calciumsilicat, Magnesiumalumosilicat, Magnesiumalumometasilicat oder ein Gemisch davon ist.

4. Pharmazeutische Zusammensetzung nach Anspruch 3, wobei das alkalische Material Natriumcarbonat, Natriumbicarbonat, Calciumsilicat, Magnesiumalumosilicat, Magnesiumalumometasilicat oder ein Gemisch davon ist.

5. Pharmazeutische Zusammensetzung nach Anspruch 4, wobei das alkalische Material ein Gemisch von Natriumcarbonat und Magnesiumalumometasilicat oder ein Gemisch von Natriumbicarbonat und Magnesiumalumometasilicat ist.

6. Pharmazeutische Zusammensetzung nach den Ansprüchen 1 bis 5, wobei der pH einer wässerigen Lösung oder Dispersionslösung der pharmazeutischen Zusammensetzung zwischen 8 und 12 liegt.

7. Pharmazeutische Zusammensetzung nach Anspruch 6, wobei der pH einer wässerigen Lösung oder Dispersionslösung der pharmazeutischen Zusammensetzung zwischen 9 und 11 liegt.

## Revendications

1. Composition pharmaceutique contenant un agent bloquant du calcium qui est de l'azelnidipine ou un sel pharmacologiquement acceptable de celui-ci et un matériel alcalin pharmacologiquement acceptable qui est un hydroxyde de métal alcalin, un hydroxyde de métal alcalino-terreux, un hydroxyde d'aluminium, un carbonate de métal alcalin, un carbonate de métal alcalino-terreux, un hydrogénocarbonate de métal alcalin, un phosphate de di-(métal alcalin), un phosphate de di-(métal alcalino-terreux), un phosphate de tri-(métal alcalin), un oxyde de métal alcalino-terreux, un oxyde d'aluminium, un silicate de métal alcalin, un silicate de métal alcalino-terreux, un composé de complexe aluminium-acide silicique, un composé de complexe aluminum-magnésium, ou un mélange de ceux-ci, dans lequel le matériel alcalin est ajouté dans une mesure telle qu'une solution aqueuse ou une solution de dispersion de ladite composition pharmaceutique contenant un agent bloquant du calcium a un pH d'au moins 8, comme déterminé par mesure avec un pH-mètre d'une solution obtenue par (i) la formation d'un mélange par dissolution ou dispersion d'une quantité de 10 fois une dose unitaire de ladite composition pharmaceutique dans 100ml d'eau distillée comme décrit dans la Pharmacopée Japonaise, (ii) la formation d'un surnageant par centrifugation dudit mélange, et (iii) la formation de ladite solution par filtration dudit surnageant.

2. Composition pharmaceutique selon la revendication 1, dans laquelle le matériel alcalin est un carbonate de métal alcalin, un carbonate de métal alcalino-terreux, un hydrogénocarbonate de métal alcalin, un oxyde de métal alcalino-terreux, un silicate de métal alcalin, un silicate de métal alcalino-terreux, un composé de complexe aluminum-magnésium, ou un mélange de ceux-ci.

3. Composition pharmaceutique selon la revendication 2, dans laquelle le matériel alcalin est du carbonate de sodium, du carbonate de magnésium, du carbonate de calcium, du bicarbonate de sodium, de l'oxyde de magnésium, de l'oxyde de calcium, du silicate de magnésium, du silicate de calcium, de l'aluminosilicate de magnésium, de l'aluminométasilicate de magnésium, ou un mélange de ceux-ci.

4. Composition pharmaceutique selon la revendication 3, dans laquelle le matériel alcalin est du carbonate de sodium, du bicarbonate de sodium, du silicate de calcium, de l'aluminosilicate de magnésium, de l'aluminométasilicate de magnésium, ou un mélange de ceux-ci.

5. Composition pharmaceutique selon la revendication 4, dans laquelle le matériel alcalin est un mélange de carbonate de sodium et d'aluminométasilicate de magnésium, ou un mélange de bicarbonate de sodium et d'aluminométasilicate de magnésium.

6. Composition pharmaceutique selon les revendications 1 à 5, dans laquelle le pH d'une solution aqueuse ou d'une solution de dispersion de ladite composition pharmaceutique est entre 8 et 12.

7. Composition pharmaceutique selon la revendication 6, dans laquelle le pH d'une solution aqueuse ou d'une solution de dispersion de ladite composition pharmaceutique est entre 9 et 11.
